(19) Europäisches Patentamt
European Patent Office
Office européen des brevets

(11) **EP 3 831 423 B1**

(12) **EUROPEAN PATENT SPECIFICATION**

(45) Date of publication and mention
of the grant of the patent:
**19.10.2022  Bulletin 2022/42**

(51) International Patent Classification (IPC):
**A61M 1/28** *(2006.01)*       **A61M 1/16** *(2006.01)*
**A61P 7/08** *(2006.01)*

(21) Application number: **19863815.7**

(22) Date of filing: **08.07.2019**

(52) Cooperative Patent Classification (CPC):
**A61M 1/287; A61M 1/1656; A61P 7/08;**
A61M 2202/02; A61M 2205/3334

(86) International application number:
**PCT/JP2019/027050**

(87) International publication number:
**WO 2020/059258 (26.03.2020 Gazette 2020/13)**

(54) **MANUFACTURING DEVICE FOR HYDROGEN-INCLUDING PERITONEAL DIALYSIS FLUID**

VORRICHTUNG ZUR HERSTELLUNG EINER WASSERSTOFFHALTIGEN
PERITONEALDIALYSEFLÜSSIGKEIT

DISPOSITIF DE FABRICATION D'UN FLUIDE DE DIALYSE PÉRITONÉALE COMPRENANT DE
L'HYDROGÈNE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority:  **21.09.2018   JP 2018176942**

(43) Date of publication of application:
**09.06.2021   Bulletin 2021/23**

(73) Proprietor: **Nihon Trim Co., Ltd.
Osaka 531-0076 (JP)**

(72) Inventor: **TACHIBANA, Takashi
Nankoku-shi, Kochi 783-0060 (JP)**

(74) Representative: **Germain Maureau
12, rue Boileau
69006 Lyon (FR)**

(56) References cited:
WO-A1-2016/063480      JP-A- 2014 161 605
JP-A- 2014 161 605      JP-A- 2015 139 475
JP-A- 2015 177 911      JP-A- 2016 013 349
JP-A- 2017 158 611

## Description

## TECHNICAL FIELD

The present invention relates to an apparatus for manufacturing hydrogen-containing peritoneal dialysate.

## BACKGROUND ART

Peritoneal dialysis has been known as one of the effective treatments for patients with renal failure who have decreased renal function and are unable to excrete urine to regulate water content and remove harmful substances inside the body including waste products such as urea.

The peritoneal dialysis is a method of removing waste products and water existing in the capillaries of the patient's peritoneum by injecting dialysate into the patient's peritoneal cavity. In the peritoneal dialysis, peritoneal dialysate having a higher osmotic pressure than body fluid (blood) is infused, and waste products and water are removed from the patient by utilizing a difference in the osmotic pressure between the body fluid and the peritoneal dialysate through the peritoneum.

Further, in recent years, it has been observed that oxidative stress occurs in dialysis patients in the peritoneal dialysis. It is considered that the oxidative stress is caused by active oxygen generated during dialysis, and it has been proposed to eliminate this active oxygen to reduce the oxidative stress.

For example, a method has been proposed according to which dialysate having a high concentration of hydrogen dissolved therein is produced by adding hydrogen molecules in dialysate enclosed in a container. Specifically, a method of adding hydrogen molecules in dialysate without opening a container is disclosed. According to the method, hydrogen molecules are brought into contact with a container (dialysate bag) having dialysate enclosed therein and having hydrogen molecule permeability from the outside of the container (the container is immersed in hydrogen-containing water, or the container is brought into contact with hydrogen gas). It is disclosed that such a method can avoid the problem of deterioration of dialysate quality due to opening of the container (see, for example, Patent Document 1).

## CITATION LIST

## PATENT DOCUMENTS

Patent Document 1: Japanese Patent No. 4486157

JP2014-161605Adiscloses a hydrogen addition device which includes: a flow passage formed of a gas permeable membrane which selectively allows hydrogen to permeate for delivering a liquid; and a hydrogen addition part for enclosing a part of the flow passage with a hydrogen transporting fluid, and adding hydrogen to the liquid by making the hydrogen contained in the hydrogen transporting fluid permeate through the gas permeable membrane.

JP2015-139475A discloses an apparatus 1 for manufacturing water for dialysis fluid preparation which includes: a tank for storing water; an electrolytic water generator connected to the tank, which is a hydrogen dissolution device for dissolving hydrogen in the water introduced from the tank; a reverse osmosis membrane processor connected to the electrolytic water generator for subjecting the water in which hydrogen is dissolved to reverse osmosis membrane treatment; and a circulation passage for circulating reverse osmosis water treated by the reverse osmosis membrane processor and introducing it into the tank.

JP2015-177911A discloses an apparatus of dialysate which comprises: an electrolytic water generation device in which hydrogen is dissolved in water; a reverse osmosis membrane processing unit connected with the electrolytic water generation device, which performs a reverse osmosis membrane processing to the water dissolved with hydrogen; a dialysate preparation device connected with the reverse osmosis membrane processing unit, which prepares dialysate where reverse osmosis water processed by the reverse osmosis membrane processing unit and dialysis undiluted solution are mixed; a dialyzer connected with the dialysate preparation device, to which the dialysate is supplied from the dialysate preparation device; a sensor arranged in the dialyzer, which detects a concentration of dissolved hydrogen in the dialysate; and a controller connected with the sensor and the electrolytic water generation device, which controls dissolution processing of hydrogen by the electrolytic water generation device based on the concentration of dissolved hydrogen detected by the sensor.

JP2016-013349A discloses a device for manufacturing hydrogen water for diluting dialysis fluid that manufactures hydrogen water for diluting dialysis fluid which includes a hydrogen supply part for supplying hydrogen gas, a gas-liquid mixer for mixing the supplied hydrogen gas with water to generate hydrogen water, a reverse osmosis membrane module arranged downstream of the gas-liquid mixer for performing filtration by a reverse osmosis membrane for the hydrogen water, a dissolved hydrogen concentration sensor arranged downstream of the gas-liquid mixer for measuring a concentration of hydrogen dissolved in the hydrogen water passing through the gas-liquid mixer, and a control part for controlling an amount of the hydrogen gas supplied by the hydrogen supply part based on the measured hydrogen concentration.

WO2016/063480A1 discloses a peritoneal dialysis fluid production apparatus which is equipped with a vessel which has hydrogen permeability and has a preset volume; a dialysis fluid which is accommodated in said vessel; a hydrogen water storage tank in which said vessel having the dialysis fluid accommodated therein is immersed and hydrogen water, which contains hydrogen and is to be supplied to the dialysis fluid, is stored; a hydrogen water production device which is connected to the hydrogen water storage tank and can produce the hydrogen water; and a circulation passage through which the hydrogen water is circulated between the hydrogen water storage tank and the hydrogen water production device.

## SUMMARY OF THE INVENTION

### TECHNICAL PROBLEM

However, the method of producing peritoneal dialysate disclosed in Patent Document 1 involves a problem in that the container needs to be taken out of the hydrogen-containing water for dialysis after the container with the dialysate enclosed therein is immersed in the hydrogen-containing water, resulting in decreased convenience.

Further, the hydrogen-containing water adheres to the outer surface of the container because the container with the dialysate enclosed therein is immersed in the hydrogen-containing water. As a result, there arises a problem that various bacteria and the like grow on the outer surface of the container with the dialysate enclosed therein, resulting in that dialysate is subject to contamination during peritoneal dialysis.

Therefore, the present invention has been made in view of the above problems, and an object of the present invention is to provide an apparatus for manufacturing hydrogen-containing peritoneal dialysate, which is excellent in terms of convenience and hygiene.

### SOLUTION TO THE PROBLEM

In order to achieve the above object, an apparatus for manufacturing hydrogen-containing peritoneal dialysate of the present invention includes a dialysate bag housing dialysate to be injected into a peritoneal cavity of a patient, and a hydrogen dissolution device interposed between the dialysate bag and the patient and configured to dissolve hydrogen in the dialysate, wherein the hydrogen dissolution apparatus includes a fluid rate measuring device connected to the dialysate bag and configured to measure a fluid rate of the dialysate supplied from the dialysate bag to the patient; a hydrogen supply amount determining device connected to the fluid rate measuring device and configured to determine an amount of hydrogen supply to the dialysate; a storage that is connected to the hydrogen supply amount determining device and stores data on a target value of the amount of hydrogen supply corresponding to the fluid rate; and a hydrogen supply device connected to the hydrogen supply amount determining device and configured to supply hydrogen to the dialysate, the hydrogen supply amount determining device is configured to determine the amount of hydrogen supply in accordance with the value of the fluid rate of dialysate measured by the fluid rate measuring device and the target value of the amount of hydrogen supply corresponding to the fluid rate of the dialysate, the target value being stored in the storage, and the hydrogen supply device is configured to supply the hydrogen to the dialysate in accordance with the amount of hydrogen supply determined by the hydrogen supply amount determining device as defined in claim 1.

### ADVANTAGES OF THE INVENTION

The present invention makes it possible to obtain peritoneal dialysate having a desired dissolved hydrogen concentration while performing peritoneal dialysis, thereby improving convenience of the peritoneal dialysis. In addition, hydrogen-containing peritoneal dialysate excellent in terms of hygiene can be obtained.

### BRIEF DESCRIPTION OF THE DRAWINGS

FIG. 1 is a schematic diagram of a configuration of an apparatus for manufacturing hydrogen-containing peritoneal dialysate according to the present invention

FIG. 2 is a diagram of a configuration of a peritoneal dialysis apparatus according to the present invention.

FIG. 3 is a flowchart for explaining a method of adjusting the concentration of a dissolved hydrogen using a hydrogen dissolution device in the apparatus for manufacturing hydrogen-containing peritoneal dialysate according to the present invention.

FIG. 4 is a schematic diagram of a configuration of an apparatus for manufacturing hydrogen-containing peritoneal dialysate according to a variation of the present invention.

### DESCRIPTION OF EMBODIMENTS

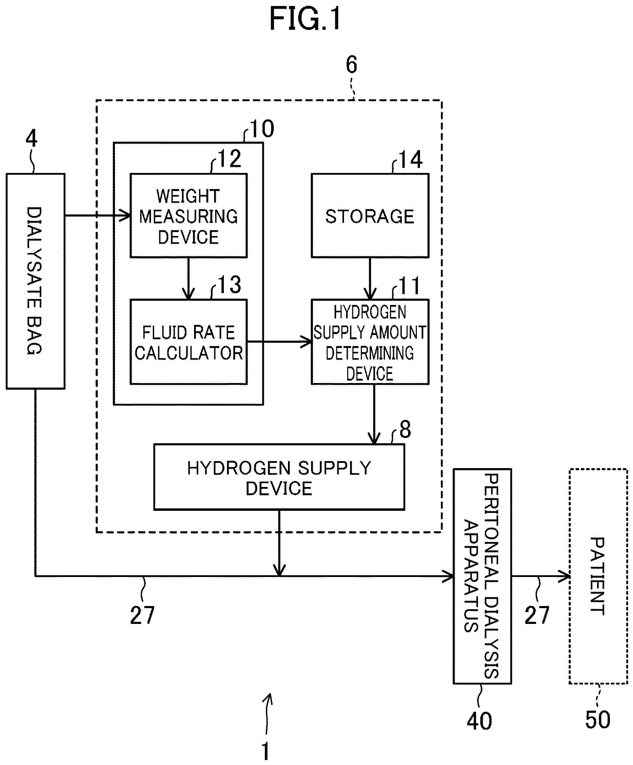

FIG. 1 is a conceptual diagram of a configuration of an apparatus for manufacturing hydrogen-containing peritoneal dialysate according to the present invention.

An apparatus 1 for manufacturing hydrogen-containing peritoneal dialysate includes a dialysate bag 4 housing dialysate 27, a peritoneal dialysis apparatus 40 connected to the dialysate bag 4 and supplied with the dialysate 27 from the dialysate bag 4, and a hydrogen dissolution device 6 interposed between the dialysate bag 4 and the peritoneal dialysis apparatus 40 and dissolving hydrogen in the dialysate 27.

The hydrogen dissolution device 6 determines the amount of hydrogen to be supplied corresponding to a fluid rate of the dialysate 27 supplied from the dialysate bag 4 to the peritoneal dialysis apparatus 40 (a flowing rate of fluid flowing per unit time) and supplies hydrogen to the dialysate 27 to maintain the concentration of dissolved hydrogen in the dialysate 27 at a desired concentration.

Specifically, as shown in FIG. 1, the hydrogen dissolution device 6 according to the present embodiment includes a fluid rate measuring device 10 connected to the dialysate bag 4 and measuring the fluid rate of the dialysate 27 supplied from the dialysate bag 4 to the peritoneal dialysis apparatus 40, and a hydrogen supply amount determining device 11 connected to the fluid rate measuring device 10 and determining the amount of hydrogen supplied to the dialysate 27. Further, the hydrogen dissolution device6 includes a storage 14 connected to the hydrogen supply amount determining device 11 and stores data on a target value of the hydrogen supply amount corresponding to the fluid rate of the dialysate 27, and a hydrogen supply device 8 connected to the hydrogen supply amount determining device 11 and supplying hydrogen to the dialysate 27 that is supplied to the peritoneal dialysis apparatus 40.

As shown in FIG. 1, the fluid rate measuring device measuring device 10 includes a weight measuring device 12 connected to the dialysate bag 4, and a fluid rate calculator 13 connected to the weight measuring device 12 and the hydrogen supply amount determining device 11.

The hydrogen supply device 8 is not particularly limited as long as it can supply hydrogen to the dialysate 27. For example, a hydrogen generator that electrolyzes water, a hydrogen generating agent such as magnesium hydride that reacts with water to generate hydrogen, a hydrogen gas cylinder, and the like can be used.

The weight measuring device 12 measures the weight of the dialysate bag 4 housing the dialysate 27. For example, a load-cell type electronic balance (manufactured by A&D Company, Limited, product name: a single point load cell LCB03) can be used, in which a load (fluid rate) is measured by amplifying a resistance change of a strain gauge attached to a load cell with an amplifier.

Unlike the conventional fluid rate sensor, such weight measuring device 12 makes it possible to measure the fluid rate without contacting the dialysate 27, which requires strict fluid quality control, and to measure the fluid rate of the dialysate 27 without contaminating the dialysate 27.

Further, using an electronic balance 12 as the weight measuring device 12 allows transmission of a signal relating to the measured weight of the dialysate bag 4 to the outside (that is, the fluid rate calculator 13).

The fluid rate calculator 13 calculates the fluid rate of the dialysate 27 supplied from the dialysate bag 4 to the peritoneal dialysis apparatus 40 in accordance with change in weight of the dialysate bag 4.

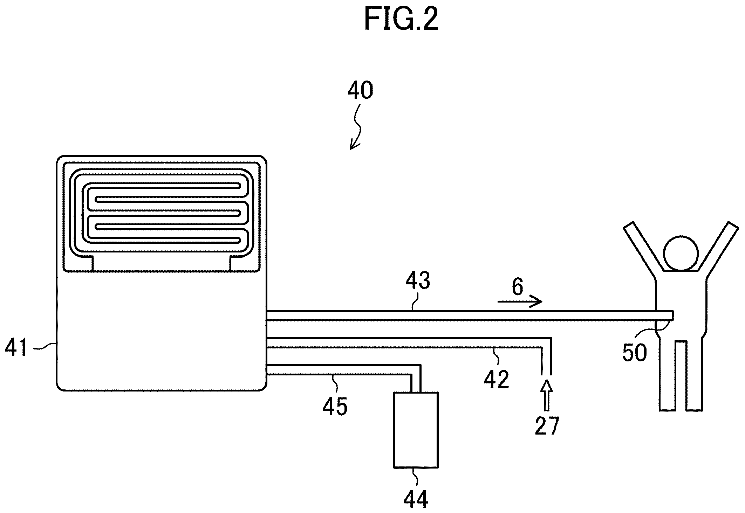

As shown in FIG. 2, the peritoneal dialysis apparatus 40 includes a dialysis apparatus body 41, a supply tube 42 into which the manufactured dialysate 27 is introduced and supplies the dialysate 27 to the dialysis apparatus body 41, and a dialysis catheter 43 connected to the dialysis apparatus body 41 and the peritoneal cavity of a patient 50 and supplying the dialysate 27 to the patient 50. Further, the peritoneal dialysis apparatus 40 includes a waste fluid treatment tank 44 for discarding unused dialysate 27, and a connecting tube 45 connecting the dialysis apparatus body 41 and the waste fluid treatment tank 44.

The dialysate 27 is supplied to the dialysis apparatus body 41 via the supply tube 42. Then, the dialysate 27 is injected into the peritoneal cavity of the patient 50 via the dialysis catheter 43 by the dialysis apparatus body 41, which is an injection treatment, and the dialysate 27 is drained from the peritoneal cavity, which is a drainage treatment.

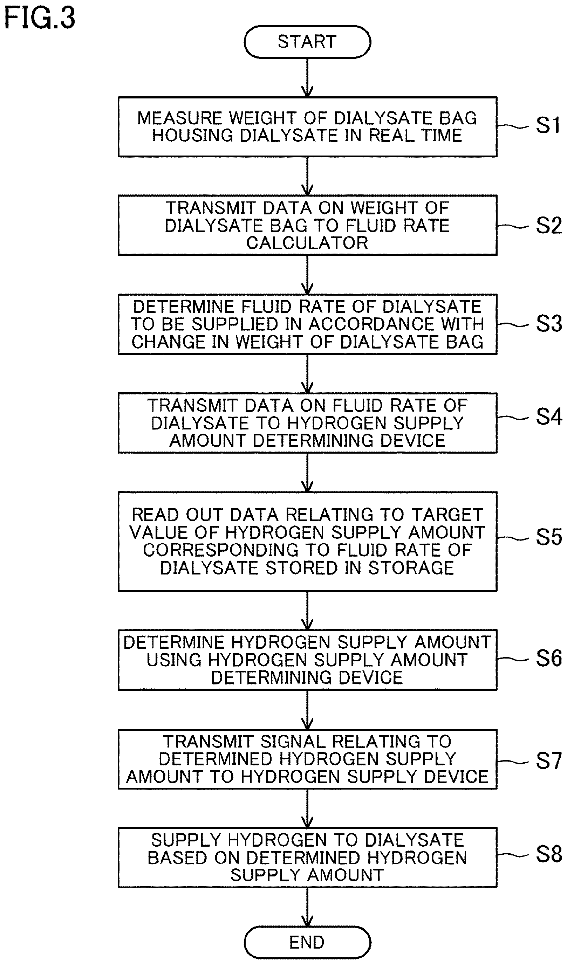

Next, a method of adjusting the concentration of dissolved hydrogen using the hydrogen dissolution device 6 will be described. FIG. 3 is a flowchart for explaining a method of adjusting the concentration of dissolved hydrogen by the hydrogen dissolution device according to the present embodiment.

First, the fluid rate measuring device 10 measures the fluid rate of the dialysate 27 supplied from the dialysate bag 4 to the peritoneal dialysis apparatus 40.

Specifically, first, during peritoneal dialysis, the weight of the dialysate bag 4 housing the dialysate 27 is measured in real time by the weight measuring device 12 (step S1), and data of the weight of the dialysate bag 4 measured in real time by the weight measuring device 12 is transmitted to the fluid rate calculator 13 (step S2).

Next, the fluid rate calculator 13 calculates the fluid rate of the dialysate 27 supplied from the dialysate bag 4 to the peritoneal dialysis apparatus 40 (flowing fluid rate per unit time) in accordance with change in input weight of the dialysate bag 4 (step S3).

Specifically, when the supply time of the dialysate 27 is T, the mass change of the dialysate bag 4 (that is, the mass of the dialysate 27 supplied from the dialysate bag 4 to the peritoneal dialysis apparatus 40) is M, and the density of the dialysate 27 is D, the fluid rate calculator 13 calculates a flowing fluid rate W of the dialysate 27 by the following equation (1).
[Mathematical 1]

$$W = M/TD \ (1)$$

Then, data of the fluid rate of the dialysate 27 calculated by the fluid rate calculator 13 is transmitted to the hydrogen supply amount determining device 11 (step S4).

Next, the hydrogen supply amount determining device 11 reads out the data stored in the storage 14 on the target value of the amount of hydrogen supply corresponding to the fluid rate of the dialysate 27 (step S5).

After that, the hydrogen supply amount determining device 11 determines the hydrogen supply amount based on the value of the fluid rate of the dialysate 27 measured by the fluid rate measuring device 10 and the target value of the amount of hydrogen supply corresponding to the fluid rate of the dialysate 27. The target value is stored in the storage 14 (step S6).

Subsequently, the hydrogen supply amount determining device 11 transmits a signal relating to the determined amount of hydrogen supply to the hydrogen supply device 8 (step S7).

Then, the hydrogen supply device 8 supplies hydrogen to the dialysate 27 based on the hydrogen supply amount determined by the hydrogen supply amount determining device 11 (step S8).

Specifically, for example, the hydrogen supply device 8 is used that includes a supply tube (not shown) to which the dialysate 27 is supplied and a housing (not shown) in which the supply tube is housed and into which hydrogen is supplied. With the dialysate 27 supplied to the inside of the supply tube, hydrogen is supplied to the outside of the supply tube inside the housing, and thus the hydrogen can be supplied to the dialysate 27.

Then, as shown in FIG. 1, the dialysate 27 supplied with hydrogen is supplied to the peritoneal dialysis apparatus 40, the dialysate 27 thus supplied is supplied from the peritoneal dialysis apparatus 40 to the patient 50, and thus the blood of the patient 50 is purified.

As described above, according to the present embodiment, the hydrogen supply amount determining device 11 determines the amount of hydrogen supply in accordance with the value of the flow rate of the dialysate 27 measured by the fluid rate measuring device 10 and the target value of the amount of hydrogen supply corresponding to the flow rate of the dialysate 27. The target value is stored in the storage 14. Further, the hydrogen supply device 8 supplies hydrogen to the dialysate 27 based on the amount of hydrogen supply determined by the hydrogen supply amount determining device 11.

Hence, this makes it possible to obtain the dialysate 27 having a desired concentration of dissolved hydrogen while performing peritoneal dialysis, thereby improving convenience of the peritoneal dialysis. In addition, the dialysate 27 excellent in terms of hygiene can be obtained.

The above embodiment may be changed as follows.

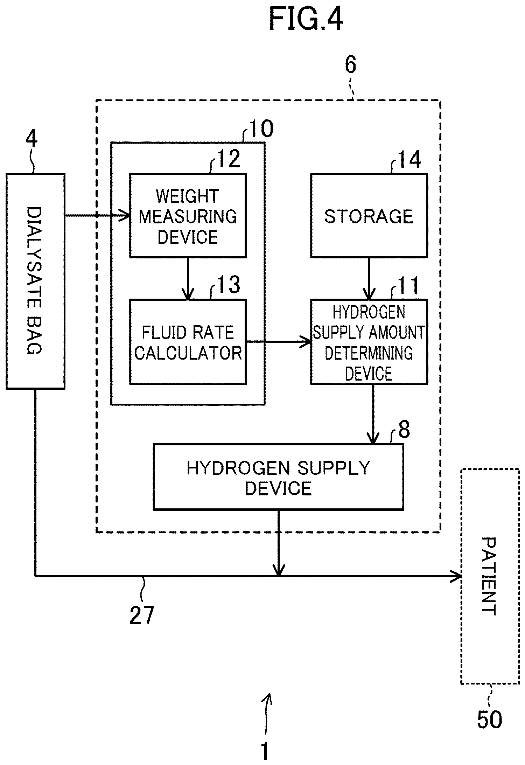

In the above embodiment, the dialysate 27 is supplied from the peritoneal dialysis apparatus 40 to the patient 50. However, as shown in FIG. 4, instead of using the peritoneal dialysis apparatus 40, the dialysate bag 4 may be connected to a tube (catheter) arranged in the patient 50, and the dialysate 27 housed in the dialysate bag 4 may be directly injected into the peritoneal cavity of the patient 50. In such a configuration, the same effect as that of the above embodiment can be obtained.

In this case, the fluid rate calculator 13 calculates the fluid rate of the dialysate 27 supplied from the dialysate bag 4 to the patient 50 in accordance with change in the weight of the dialysate bag 4 measured by the weight measuring device 12 (that is, the flowing fluid rate of the dialysate 27 supplied from the dialysate bag 4 to the patient 50 per unit time).

INDUSTRIAL APPLICABILITY

As described above, the present invention is particularly useful for an apparatus for manufacturing hydrogen-containing peritoneal dialysate.

DESCRIPTION OF REFERENCE CHARACTERS

| | |
|---|---|
| 1 | Apparatus for Manufacturing Hydrogen-Containing Peritoneal Dialysate |
| 4 | Dialysate Bag |
| 6 | Hydrogen Dissolution Device |
| 8 | Hydrogen Supply Device |
| 10 | Fluid Rate Measuring Device |
| 11 | Hydrogen Supply Amount Determining Device |
| 12 | Weight Measuring Device |
| 13 | Fluid Rate Calculator |
| 14 | Storage |
| 27 | Dialysate |
| 40 | Peritoneal Dialysis Apparatus |
| 50 | Patient |

**Claims**

1. An apparatus (1) for manufacturing hydrogen-containing peritoneal dialysate (27), the apparatus comprising:

   a dialysate bag (4) housing dialysate (27) to be injected into a peritoneal cavity of a patient; and
   a hydrogen dissolution device (6) interposed between the dialysate bag (4) and the patient and configured to dissolve hydrogen in the dialysate (27),
   the hydrogen dissolution device (6) including:

   a fluid rate measuring device (10) connected to the dialysate bag (4) and configured to measure a flow rate of the dialysate (27) supplied from the dialysate bag (4) to the patient;
   a hydrogen supply amount determining device (11) connected to the fluid rate measuring device (10) and configured to determine an amount of hydrogen supply to the dialysate (27);
   a storage (14) connected to the hydrogen supply amount determining device (11) and configured to store data on a target value of the hydrogen supply amount corresponding to the fluid rate of the dialysate (27); and a hydrogen supply device (8) connected to the hydrogen supply amount determining device (11) and configured to supply hydrogen to the dialysate (27), wherein

the hydrogen supply amount determining device (11) is configured to determine the amount of hydrogen supply in accordance with a value of the fluid rate of the dialysate (27) measured by the fluid rate measuring device (10) and the target value of the amount of hydrogen supply corresponding to the fluid rate of the dialysate (27), the target value being stored in the storage, the hydrogen supply device (8) is configured to supply the hydrogen to the dialysate (27) in accordance with the amount of hydrogen supply determined by the hydrogen supply amount determining device (11), the fluid rate measuring device (10) includes a weight measuring device (12) connected to the dialysate bag (4) and a fluid rate calculator (13) connected to the weight measuring device (12), the weight measuring device (12) is configured to measure a weight of the dialysate bag (4) housing the dialysate (27) in real time, the fluid rate calculator (13) is configured to calculate the fluid rate of the dialysate (27) supplied from the dialysate bag (4) to the patient in accordance with change in the weight of the dialysate bag (4) measured by the weight measuring device (12), and the hydrogen supply amount determining device (11) is configured to determine the hydrogen supply amount in accordance with the value of the fluid rate of the dialysate (27) calculated by the fluid rate calculator (13) and the target value of the amount of hydrogen supply corresponding to the fluid rate of the dialysate (27), the target value being stored in the storage (14).

2. The apparatus for manufacturing hydrogen-containing peritoneal dialysate (27) of claim 1, wherein the fluid rate calculator (13) is configured to calculate a flowing fluid rate of the dialysate (27) supplied from the dialysate bag (4) to the patient per unit time.

3. The apparatus for manufacturing hydrogen-containing peritoneal dialysate (27) of claim 1 or 2, wherein the weight measuring device (12) is an electronic balance.

**Patentansprüche**

1. Einrichtung (1) zur Herstellung von wasserstoffhaltigem Peritoneal-Dialysat (27), wobei die Einrichtung umfasst:

einen Dialysatbeutel (4), der Dialysat (27) beinhaltet, welches in eine Peritonealhöhle eines Patienten injiziert werden soll; und eine Wasserstoff-Auflösevorrichtung (6), die zwischen dem Dialysatbeutel (4) und dem Patienten eingefügt und dazu ausgelegt ist, Wasserstoff im Dialysat (27) aufzulösen, wobei die Wasserstoff-Auflösevorrichtung (6) einschließt:

eine Fluidraten-Messvorrichtung (10), die mit dem Dialysatbeutel (4) verbunden und dazu ausgelegt ist, eine Strömungsrate des Dialysats (27), welches dem Patienten aus dem Dialysatbeutel (4) zugeführt wird, zu messen; eine Wasserstoffzufuhrmengen-Ermittlungsvorrichtung (11), die mit der Fluidraten-Messvorrichtung (10) verbunden und dazu ausgelegt ist, eine Menge der Wasserstoffzufuhr zum Dialysat (27) zu ermitteln; einen Speicher (14), der mit der Wasserstoffzufuhrmengen-Ermittlungsvorrichtung (11) verbunden und dazu ausgelegt ist, Daten über einen Zielwert der Wasserstoffzufuhrmenge, welcher der Fluidrate des Dialysats (27) entspricht, zu speichern; und eine Wasserstoffzufuhrvorrichtung (8), die mit der Wasserstoffzufuhrmengen-Ermittlungsvorrichtung (11) verbunden und dazu ausgelegt ist, dem Dialysat (27) Wasserstoff zuzuführen, wobei die Wasserstoffzufuhrmengen-Ermittlungsvorrichtung (11) dazu ausgelegt ist, die Menge der Wasserstoffzufuhr in Übereinstimmung mit einem von der Fluidraten-Messvorrichtung (10) gemessenen Wert der Fluidrate des Dialysats (27) und dem Zielwert der Wasserstoffzufuhrmenge, welcher der Fluidrate des Dialysats (27) entspricht, zu ermitteln, wobei der Zielwert im Speicher gespeichert ist, die Wasserstoffzufuhrvorrichtung (8) dazu ausgelegt ist, dem Dialysat (27) den Wasserstoff in Übereinstimmung mit der von der Wasserstoffzufuhrmengen-Ermittlungsvorrichtung (11) ermittelten Wasserstoffzufuhrmenge zuzuführen, die Fluidraten-Messvorrichtung (10) eine Gewichtsmessvorrichtung (12), die mit dem Dialysatbeutel (4) verbunden ist, und einen Fluidraten-Rechner (13), der mit der Gewichtsmessvorrichtung (12) verbunden ist, einschließt,

die Gewichtsmessvorrichtung (12) dazu ausgelegt ist, ein Gewicht des Dialysatbeutels (4), der das Dialysat (27) beinhaltet, in Echtzeit zu messen,

der Fluidraten-Rechner (13) dazu ausgelegt ist, die Fluidrate des dem Patienten aus dem Dialysatbeutel (4) zugeführten Dialysats (27) in Übereinstimmung mit einer von der Gewichtsmessvorrichtung (12) gemessenen Veränderung des Gewichts des Dialysatbeutels (4) zu berechnen, und

die Wasserstoffzufuhrmengen-Ermittlungsvorrichtung (11) dazu ausgelegt ist, die Wasserstoffzufuhrmenge in Übereinstimmung mit dem vom Fluidraten-Rechner (13) berechneten Wert der Fluidrate des Dialysats (27) und dem Zielwert der Wasserstoffzufuhrmenge, welcher der Fluidrate des Dialysats (27) entspricht, zu ermitteln, wobei der Zielwert im Speicher (14) gespeichert ist.

2. Einrichtung zur Herstellung von wasserstoffhaltigem Peritoneal-Dialysat (27) nach Anspruch 1, wobei der Fluidraten-Rechner (13) dazu ausgelegt ist, eine Fluidströmungsrate des Dialysats (27), das dem Patienten aus dem Dialysebeutel (4) zugeführt wird, pro Zeiteinheit zu berechnen.

3. Einrichtung zur Herstellung von wasserstoffhaltigem Peritoneal-Dialysat (27) nach Anspruch 1 oder 2, wobei
es sich bei der Gewichtsmessvorrichtung (12) um eine elektronische Waage handelt.

**Revendications**

1. Appareil (1) pour fabriquer un dialysat péritonéal contenant de l'hydrogène (27), l'appareil comprenant :

une poche de dialysat (4) recevant un dialysat (27) à injecter dans une cavité péritonéale d'un patient ; et
un dispositif de dissolution d'hydrogène (6) interposé entre la poche de dialysat (4) et le patient et configuré pour dissoudre l'hydrogène dans le dialysat (27),
le dispositif de dissolution d'hydrogène (6) comprenant :

un dispositif de mesure de débit de fluide (10) relié à la poche de dialysat (4) et configuré pour mesurer un débit du dialysat (27) fourni depuis la poche de dialysat (4) au patient ;
un dispositif de détermination de quantité d'alimentation en hydrogène (11) relié au dispositif de mesure de débit de fluide (10) et configuré pour déterminer une quantité d'alimentation en hydrogène du dialysat (27) ;
une mémoire (14) reliée au dispositif de détermination de quantité d'alimentation en hydrogène (11) et configurée pour stocker des données sur une valeur cible de la quantité d'alimentation en hydrogène correspondant au débit de fluide du dialysat (27) ; et
un dispositif d'alimentation en hydrogène (8) relié au dispositif de détermination de quantité d'alimentation en hydrogène (11) et configuré pour alimenter le dialysat (27) en hydrogène, dans lequel
le dispositif de détermination de quantité d'alimentation en hydrogène (11) est configuré pour déterminer la quantité d'alimentation en hydrogène conformément à une valeur du débit de fluide du dialysat (27) mesuré par le dispositif de mesure de débit de fluide (10) et à la valeur cible de la quantité d'alimentation en hydrogène correspondant au débit de fluide du dialysat (27), la valeur cible étant stockée dans la mémoire,
le dispositif d'alimentation en hydrogène (8) est configuré pour alimenter le dialysat (27) en hydrogène conformément à la quantité d'alimentation en hydrogène déterminée par le dispositif de détermination de quantité d'alimentation en hydrogène (11),
le dispositif de mesure de débit de fluide (10) comprend un dispositif de mesure de poids (12) relié à la poche de dialysat (4) et un calculateur de débit de fluide (13) relié au dispositif de mesure de poids (12),
le dispositif de mesure de poids (12) est configuré pour mesurer un poids de la poche de dialysat (4) recevant le dialysat (27) en temps réel,
le calculateur de débit de fluide (13) est configuré pour calculer le débit de fluide du dialysat (27) fourni depuis la poche de dialysat (4) au patient conformément au changement du poids de la poche de dialysat (4) mesuré par le dispositif de mesure de poids (12), et
le dispositif de détermination de quantité d'alimentation en hydrogène (11) est configuré pour déterminer la quantité d'alimentation en hydrogène conformément à la valeur du débit de fluide du dialysat (27) calculé par le calculateur de débit de fluide (13) et à la valeur cible de la quantité d'alimentation en hydrogène correspondant au débit de fluide du dialysat (27), la valeur cible étant stockée dans la mémoire (14).

2.  Appareil pour fabriquer un dialysat péritonéal contenant de l'hydrogène (27) de la revendication 1, dans lequel
    le calculateur de débit de fluide (13) est configuré pour calculer un débit de fluide en écoulement du dialysat (27) fourni depuis la poche de dialysat (4) au patient par unité de temps.

3.  Appareil pour fabriquer un dialysat péritonéal contenant de l'hydrogène (27) de la revendication 1 ou 2, dans lequel
    le dispositif de mesure de poids (12) est une balance électronique.

# FIG.1

# FIG.2

EP 3 831 423 B1

# FIG.3

START

MEASURE WEIGHT OF DIALYSATE BAG HOUSING DIALYSATE IN REAL TIME — S1

TRANSMIT DATA ON WEIGHT OF DIALYSATE BAG TO FLUID RATE CALCULATOR — S2

DETERMINE FLUID RATE OF DIALYSATE TO BE SUPPLIED IN ACCORDANCE WITH CHANGE IN WEIGHT OF DIALYSATE BAG — S3

TRANSMIT DATA ON FLUID RATE OF DIALYSATE TO HYDROGEN SUPPLY AMOUNT DETERMINING DEVICE — S4

READ OUT DATA RELATING TO TARGET VALUE OF HYDROGEN SUPPLY AMOUNT CORRESPONDING TO FLUID RATE OF DIALYSATE STORED IN STORAGE — S5

DETERMINE HYDROGEN SUPPLY AMOUNT USING HYDROGEN SUPPLY AMOUNT DETERMINING DEVICE — S6

TRANSMIT SIGNAL RELATING TO DETERMINED HYDROGEN SUPPLY AMOUNT TO HYDROGEN SUPPLY DEVICE — S7

SUPPLY HYDROGEN TO DIALYSATE BASED ON DETERMINED HYDROGEN SUPPLY AMOUNT — S8

END

# FIG.4

**EP 3 831 423 B1**

**REFERENCES CITED IN THE DESCRIPTION**

**Patent documents cited in the description**

- JP 4486157 B
- JP 2014161605 A
- JP 2015139475 A
- JP 2015177911 A
- JP 2016013349 A
- WO 2016063480 A1